(19) Europäisches Patentamt
European Patent Office
Office européen des brevets

(11) Veröffentlichungsnummer: **0 199 210 B1**

(12) **EUROPÄISCHE PATENTSCHRIFT**

(45) Veröffentlichungstag der Patentschrift: **16.10.91**

(51) Int. Cl.5: **C07C 45/60**, C07C 47/198, C07C 47/277, C07C 47/37, C07D 521/00, //C07D307/12, C07D307/42,C07D333/16, C07D213/30

(21) Anmeldenummer: **86104968.2**

(22) Anmeldetag: **11.04.86**

(54) **Umwandlung von 1,3-Dioxanen zu 4-Oxa-aldehyden.**

(30) Priorität: **17.04.85 DE 3513725**

(43) Veröffentlichungstag der Anmeldung:
**29.10.86 Patentblatt 86/44**

(45) Bekanntmachung des Hinweises auf die Patenterteilung:
**16.10.91 Patentblatt 91/42**

(84) Benannte Vertragsstaaten:
**BE CH DE FR GB IT LI NL**

(56) Entgegenhaltungen:
**DE-A- 2 922 698**
**FR-A- 1 267 507**
**US-A- 2 561 254**
**US-A- 3 676 500**

**JACS, Band 84, 5. September 1962, Seiten 3307-3319; C.S. RONDESTVEDT, Jr. et al.: "A new rearrangement. Catalytic isomerization of m-Dioxanes to beta-alkoxy aldehydes. II. Scope and limitations"**

**CHEMICAL ABSTRACTS, Band 93, 1980, Seite 638, Zusammenfassung Nr. 132246q, Columbus, Ohio, US**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18c**
**W-6710 Frankenthal(DE)**
Erfinder: **Merger, Franz, Dr.**
**Max-Slevogt-Strasse 25**
**W-6710 Frankenthal(DE)**
Erfinder: **Fischer, Rolf, Dr.**
**Bergstrasse 98**
**W-6900 Heidelberg(DE)**

**EP 0 199 210 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Vorliegende Anmeldung betrifft ein Verfahren zur Herstellung von 4-Oxa-aldehyden durch katalytische Isomerisierung Von 1,3-Dioxanen.

Es ist bekannt, daß 1,3-Dioxan und dessen Derivate zu $\beta$-Alkoxyaldehyden umgelagert werden können (J. Am. Chem. Soc. 892 (1960), 6419-6420 und J. Am. Chem. Soc. 84 (1962), 3307-3319 u. 3319-3326). Als Katalysatoren wurden hierbei Silicagel und Bimsstein eingesetzt. Die verwendeten Katalysatoren zeigen starke Desaktivierungserscheinungen. Auch sind ihre Aktivität und Selektivität wirtschaftlich noch nicht befriedigend. Wegen der unzureichenden Spezifizierung des Naturproduktes Bimsstein, das je nach Herkunft unterschiedliche Zusammensetzung aufweisen kann, kann es leicht zu unkontrollierbaren Einflüssen auf die Reaktion kommen (Houben Weyl, Methoden d. org. Chemie IV, 2, S. 149 (1955)).

Ferner ist die Umwandlung von 1,3-Dioxanen zu 4-Oxa-aldehyden durch katalytische Isomerisierung

a) mit Bimsstein (US-A- 3,676,500) oder

b) mit Montmorillonit in der H-Form (FR-A- 1,267,507) bekannt.

Desweiteren ist aus Chem. Abstracts Vo1. 93, 132 246 q (1980) die Herstellung von Benzyloxytrimethylacetaldehyd durch Gasphasenisomerisierung von 5,5-Dimethyl-2-phenyl-1,3-dioxan an Bimsstein bekannt.

In der DE-OS 29 22 698 wird ein Verfahren zur Herstellung von $\beta$-Alkoxypivalinaldehyd aus 1,3-Dioxanen unter Verwendung von Siliciumdioxid, dotiert mit Hydroxiden der Gruppe III A und/oder III B und Alkalihydroxid, als Katalysator beschrieben. Diese Katalysatoren, die aber nur einen graduellen Fortschritt bringen, unterscheiden sich also von den vorher beschriebenen durch Neutralisation der aciden Zentren. Die für die Katalysatorfertigung notwendigen Verbindungen der reinen Lanthanide Praseodym und Neodym sind teure und nicht leicht zugängliche Chemikalien. Das bevorzugt benutzte handelsübliche Lanthaniden-Gemisch Didymium variiert in seiner Zusammensetzung, so daß die Reproduzierung der technischen Katalysatoren schwierig ist. Die angegebenen Katalysatorstandzeiten liegen nur im Stundenbereich; über die Regenerierung der Katalysatoren wird keine Aussage gemacht.

Der Erfindung lag nun die Aufgabe zugrunde, ein Verfahren zu entwickeln, wonach bekannte aber auch bisher nicht zugängliche 4-Oxa-aldehyde aus den entsprechenden 1,3-Dioxanen hergestellt werden können und wobei sich die dazu nötigen Katalysatoren durch einfache Verfügbarkeit, hohe Aktivität und leichte Regenerierbarkeit auszeichnen sollten. Weiterhin sollten bei langen Katalysatorstandzeiten hohe Umsätze und Selektivitäten gewährleistet sein.

Demgemäß wurde ein Verfahren zur Herstellung von 4-Oxa-aldehyden der Formel (I)

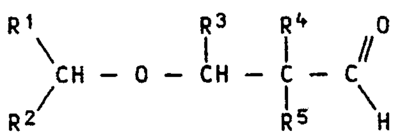

(I)

durch katalytische Isomerisierung von 1,3-Dioxanen gefunden, das sich dadurch auszeichnet, daß man 1,3-Dioxane der Formel (II)

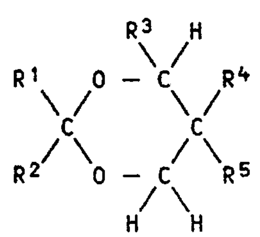

(II),

wobei die Reste $R^1$, $R^2$, $R^4$ und $R^5$ in den Formeln (I) und (II) untereinander gleich oder verschieden sind und für Wasserstoff, geradkettige oder verzweigte Alkyl-, Alkenyl oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder für heterocyclische Reste stehen und darüber hinaus jeweils die Reste $R^1$ und $R^2$ und/oder $R^4$ und $R^5$ zusammen mit dem C-Atom an das sie gebunden sind, ein Cycloalkan, Cycloalken oder einen Heterocyclus mit 5 bis 7 Ringgliedern bilden können, wobei die genannten Reste noch unter den Reaktionsbedingungen inerte Substituenten tragen können und wobei der Rest $R^3$ Wasserstoff oder einen geradkettigen oder verzweigten Alkylrest bedeutet, unter

Verwendung von aciden zeolithischen Katalysatoren isomerisiert.

Im erfindungsgemäßen Verfahren werden die eingangs an die Katalysatoren gestellten Anforderungen weitgehend erfüllt. Im Hinblick auf den Stand der Technik ist das Gelingen des Verfahrens besonders überraschend, weil die zuvor begangene Linie gerade in entgegengesetzter Richtung, nämlich der Ausschaltung von aciden Zentren, wies. Daher hat man nicht erwartet, daß gerade mit Zeolithen, die sich durch besonders hohe Acidität sowie durch strenge Strukturparameter auszeichnen, in so weiten Grenzen so hervorragende Ergebnisse erhalten werden.

Die Umwandlung dieser 1,3-Dioxane zu 4-Oxa-aldehyden bietet eine gute Methode, um beispielsweise die Ether der Hydroxyneoalkanale in hoher Selektivität bei hohem Umsatz herzustellen, die nach konventionellen Veretherungsmethoden nicht bzw. nur schwierig zugänglich sind.

Die Umwandlung läßt sich durch folgende Gleichung wiedergeben:

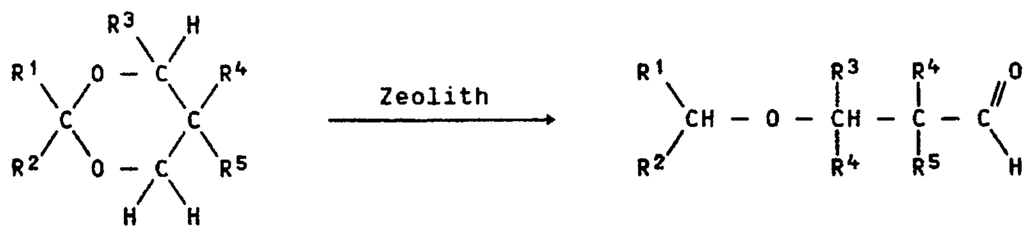


(II) (I)

Die als Ausgangsstoffe verwendeten 1,3-Dioxane der Formel (II) und dementsprechend die entstehenden 4-Oxa-aldehyde der Formel (I) enthalten die Reste $R^1$, $R^2$, $R^4$ und $R^5$, die gleich oder verschieden sind und unabhängig voneinander für Wasserstoff, verzweigte oder unverzweigte, Alkyl-, Alkenyl-oder Alkinylreste mit bis zu 18 Kohlenstoffatomen, insbesondere 1 bis 12, vorzugsweise 1 bis 6 Kohlenstoffatomen, Cycloalkyl- oder Cycloalkenylreste mit 5 bis 8, insbesondere 5 und 6 Kohlenstoffatomen, Aryl-, Alkylaryl-, Aralkyl-, Aralkenyl- oder Alkenylarylreste mit 6 bis 16, insbesondere 6 bis 12 Kohlenstoffatomen, aromatische, gesättigte und ungesättigte Heterocyclen, die ein oder mehrere Heteroatome wie Stickstoff, Sauerstoff oder Schwefel enthalten.

Die genannten Reste können noch unter den Reaktionsbedingungen inerte Substituenten tragen. So können z.B. die aus Verbindung (II) entstandenen Produkte (I) ihrerseits mit einem Diol über ein Acetal der allgemeinen Formel (IV)

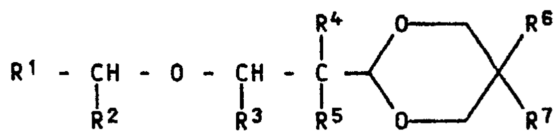

(IV),

in der die Reste $R^6$ und $R^7$ im oben angegebenen Definitionsbereich von $R^4$ und $R^5$ liegen, zu Aldehyden der Struktur (III)

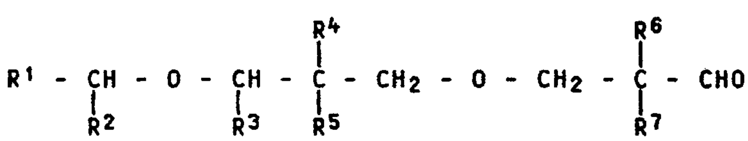

(III)

weiter aufgebaut werden.

Die Reste $R^1$ und $R^2$ und/oder $R^4$ und $R^5$ und/oder $R^6$ und $R^7$ können auch zusammen mit dem C-Atom an das sie gebunden sind ein Cycloalkan, Cycloalken oder einen Heterocyclus bilden.

Als Rest $R^3$ kommen unabhängig von den übrigen Resten Wasserstoff sowie geradkettige oder verzweigte Alkylreste mit 1 bis 12, insbesondere 1 bis 8, vorzugsweise 1 bis 4 Kohlenstoffatomen in Betracht.

Alkyl-, Alkenyl- oder Alkinylreste sind z.B. Methyl-, Ethyl-, n-Propyl-, i-Propyl-, Propenyl-, i-Propenyl-, n-Butyl, i-Butyl-, n-Butenyl-, i-Butenyl-, n-Butinyl-, Pentyl-, Pentenyl-, Pentinyl-, Hexyl-, Hexenyl-, Heptyl-, Heptenyl-, Octyl-, Octenyl-, Nonyl-, Nonenyl-, Decyl-, Decenyl-, Dodecyl- oder Dodecenyl-Reste. Die Alkyl-, Alkenyl- oder Alkinylreste können noch unter den Reaktionsbedingungen inerte Substituenten tragen, z.B. Halogen, Alkoxy-, Carboxy- oder Carboxyatgruppen.

Cycloalkylreste sind z.B. Cyclopentyl-, Cyclohexyl-, Cycloheptyl-, Cyclopentenyl- oder Cyclohexenylreste.

Als aromatische Reste kommen z.B. Phenyl-, Benzyl-, 2-Phenylethyl-, 3-Phenylpropyl-, 2-Phenylpropyl-, 4-Phenylbutyl-, 3-Phenylbutyl-, 2-Phenylbutyl- oder 3-Phenylbutenyl-Reste in Betracht, die gegebenenfalls noch durch unter den Reaktionsbedingungen inerte Reste substituiert sein können.

Heterocyclische bzw. heteroaromatische Reste sind z.B. Tetrahydrofuran-, Dihydrofuran-, Furan-, Tetrahydrothiophen(Thiophan)-, Dihydrothiophen-, Thiophen-, Pyridin-, Thiopyran. Diese Reste können noch durch unter den Reaktionsbedingungen inerte Reste wie Alkylreste oder Halogenatome substituiert sein.

Für das erfindungsgemäße Verfahren besonders geeignete Ausgangsstoffe sind solche 1,3-Dioxane, in denen $R^3$ Wasserstoff bedeutet, während $R^4$ und $R^5$ einen der genannten organischen Reste darstellen.

Die Ausgangsverbindungen der Formel (II) lassen sich nach bekannten Methoden aus Aldehyden oder Ketonen oder deren leicht spaltbaren Derivaten, z.B. Dialkylketalen oder Acetalen mit 1,3-Diolen gemäß folgender Reaktionsgleichung herstellen.

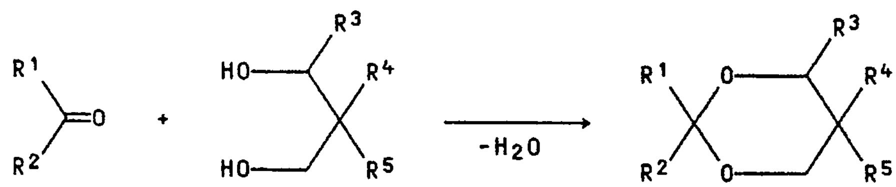


Als Diolkomponente kommen z.B. folgende Verbindungen in Betracht: Propandiol-1,3, 2-Methyl-, 2-Ethyl-, 2-Phenyl-, 2,2-Dimethyl-, 2,2-Diethyl-, 2-Methyl-2-ethyl-, 2-Methyl-2-propyl-, 2-Methyl-2-butyl-, 2-Methyl-2-phenyl und 2-Ethyl-2-butyl-propandiol-1,3, 1,1-Dimethylolcyclohexan und -pentan, 3,3-Dimethyloltetrahydrofuran und -pyran sowie 2,2,4-Trimethyl-pentandiol-1,3.

Geeignete Carbonylkomponenten sind z.B. aliphatische, aromatische oder heterocyclische Aldehyde und Ketone oder deren Acetale bzw. Ketale mit niedrig siedenden Alkoholen.

Gesättigte aliphatische Aldehyde sind z.B. Formaldehyd, Acet-, Propion-oder Butyraldehyd, Pentanal, Hexanal und höhere homologe n-Alkanale wie Dekanal. Isobutyraldehyd, 2-Methylbutanal, 3-Methylbutanal, 3,3-Dimethylbutanal, 2-Methylpentanal, 2-Ethylhexanal und 2-Methyldekanal. Glyoxal, Methylglyoxal, Malondialdehyd, Succindialdehyd und Glutardialdehyd.

Heterocyclische Aldehyde sind z.B. Tetrahydrofuryl-2-aldehyd und -3-aldehyd, Tetrahydrothienyl-2- und -3-aldehyd, 5,6-Dihydropyranyl-6-aldehyd, 2,5-Dimethyl-5,6-dihydropyranyl-6-aldehyd, Furyl-2-aldehyd und -3-aldehyd, Thienyl-3-aldehyd, 2-, 3- oder 4-Pyridylaldehyd.

Als Ketone kommen beispielsweise die folgenden Verbindungen in Betracht: Aceton, Methylethylketon, Diethylketon, Methylisopropylketon, Diisopropyl-und Diisobutylketon, Methylisobutylketon, Methoxyaceton, Methylvinylketon, Methylisopropenyl- und Methylisobutenylketon, Cyclopentanon, Cyclohexanon, Methylcyclopentanone, Methylcyclohexanone, Cyclohexenon, 3,5,5-Trimethylcyclohexenon-2, Methyl-, Ethyl- und Vinylphenylketon, Methylfurylketon, Acetylaceton und Acetessigester.

Die oben genannten Verbindungen stellen eine Auswahl verwendbarer Komponenten zur Herstellung von substituierten 1,3-Dioxanen dar und sollen die Anwendungsbreite des erfindungsgemäßen Verfahrens auf eine Vielzahl von 1,3-Dioxanen nicht einschränken.

Weitere substituierte Alkanale sind beispielsweise 3-Hydroxy-2,2-dimethylpropanal (Hydroxypivalinaldehyd), Methoxy- und Butoxypivalinaldehyd, 4-Acetoxybutyraldehyd und 5-Formylvaleriansäureethylester.

Weiterhin können ungesättigte Aldehyde verwendet werden, z.B. Acrolein, α-Methylacrolein, α-Ethylacrolein und höhere α-Alkyl-, Isoalkyl- und Alkenylacroleine, But-2-enal, But-3-enal, 2-Methyl-but-2-enal, 2-Methylpent-2-enal, 2-Ethyl-hex-2-enal, 2,2-Dimethyl-pent-4-enal, 2-Methyl-4-acetoxy-but-2-enal, 2-Methoxymethylacrolein, 2-(3-Methoxycarbonylpropyl)-acrolein oder 2-Methyl-4-chlor-but-2-enal.

Aromatische Aldehyde sind z.B. Benzaldehyd, p-Methoxybenzaldehyd, Phenylacetaldehyd, 2-Phenyl und 3-Phenylpropanal, 2-Hydroxybenzaldehyd, 3-Hydroxy-4-methoxybenzaldehyd, Zimtaldehyd oder Benzylacrolein.

Als Katalysatoren für die erfindungsgemäße Umwandlung von 1,3-Dioxanen werden acide zeolithische

Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt.

In den Zeolithen können anstelle von Aluminium auch andere Elemente wie B, Ga, Fe, Cr, V, As, Sb in das Gitter eingebaut werden oder das Silicium kann durch ein vierwertiges Element wie Ge ersetzt werden.

Als Katalysatoren kommen Zeolithe aus der Faujasit-Gruppe, z.B. der Zeolith Y oder aus der Mordenit-Gruppe oder engporige Zeolithe z.B. vom Erionit- bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzung aufweisen. Es handelt sich hierbei um Alumino-, Boro-, Eisen-, Gallium-, Chrom-, Arsen-, Antimon-und Bismutsilikatzeolithe oder deren Gemische sowie um Alumino-, Boro-, Gallium- und Eisengermanatzeolithe oder deren Gemische.

Insbesondere eignen sich die Alumino-, Boro- und Eisensilikatzeolithe des Pentasiltyps für die erfindungsgemäße Isomerisierung. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit oder ohne Alkali- oder Erdalkalizusatz bei 100 bis 220 °C unter autogenem Druck hergestellt. Die erhaltenen Aluminosilikatzeolithe enthalten je nach Wahl der Einsatzstoffmengen ein $SiO_2/Al_2O_3$-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw. Boro- bzw. Eisensilikatzeolith direkt nach der Trocknung verformt wird und erstmals nach der Verformung einer Calcinierung unterworfen wird. Die hergestellten Alumino-, Boro- und Eisensilikatzeolithe können aber auch in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht

z.B. darin, daß man den unverformten Zeolithen mit Metallsalzen durch einen Ionenaustausch oder durch Imprägnierung dotiert.

Insbesondere durch eine Dotierung der Zeolithe mit Übergangsmetallen wie Mo, Fe, Zn, Cu und vor allem W, mit Edelmetallen wie Pd und mit seltenen Erdmetallen wie La werden vorteilhafte Katalysatoren erhalten.

Zweckmäßigerweise führt man die Dotierung so durch, daß man z.B. den verformten Pentasilzeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Wolframsäure $H_2WO_4$ in Wasser - größtenteils zumindest - löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z.B. eine ammoniaklische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniaklische $Pd(NO_3)_2$-Lösung bei leicht erhöhter Temperatur zwischen 30 und 80 °C im Kreislauf 15 bis 20 h leitet. Danach wird mit Wasser ausgewaschen, bei ca. 150 °C getrocknet und bei ca. 550 °C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Die hier beschriebenen Katalysatoren können wahlweise als Stränge, Tabletten, Pulver oder als Wirbelkontakt eingesetzt werden. Vorteilhaft können 2 bis 4mm lange Stränge, Tabletten mit einem Durchmesser von 3 bis 5mm und Pulver bzw. Wirbelkontakte mit Teilchengrößen von 0,1 bis 0,5mm verwendet werden.

Die für die erfindungsgemäße Umwandlung in der Regel gewählten Reaktionsbedingungen sind in der Gasphase 200 bis 500 °C, vorzugsweise 230 bis 400 °C, und eine Belastung WHSV = 0,1 bis $20 h^{-1}$, bevorzugt 0,5 bis $5 h^{-1}$ (g 1,3-Dioxan/g Katalysator und Stunde). Im allgemeinen steigt der Umsatz mit steigender Temperatur stark an, während die Selektivität in einen bestimmten Temperaturbereich nur wenig zurückgeht.

Das Verfahren wird in der Regel bei Normaldruck oder je nach Flüchtigkeit der Ausgangsverbindung bei vermindertem oder erhöhtem Druck durchgeführt, wobei die Durchführung vorzugsweise kontinuierlich erfolgt.

Nach der Umsetzung werden die entstandenen 4-Oxa-aldehyde nach den dafür üblichen Techniken z.B. durch Destillation aus dem Reaktionsgemisch isoliert; nichtumgesetzte 1,3-Dioxane (II) werden gegebenenfalls für die erfindungsgemäße Umsetzung zurückgeführt.

Die nach dem erfindungsgemäßen Verfahren zugänglichen Verbindungen selber und eine Reihe ihrer Derivate sind als biologisch aktive Verbindungen, z.B. als Bakterizide von Interesse und darüber hinaus wertvolle Zwischenprodukte. Sie lassen sich z.B. leicht zu Aminen, Alkoholen und Säuren nach dem Fachmann geläufigen Methoden, z.B. durch Oxidation mit Sauerstoff oder durch Reduktion z.B. durch katalytische Hydrierung oder aminierende Hydrierung weiterverarbeiten. Die Überführung der erfindungsgemäßen Ether in tert.-Butylperoxyester z.B. nach dem in der DE-OS 29 22 698 beschriebenen Verfahren führt zu vorteilhaften Polymerisationsinitiatoren.

Die folgenden Beispiele veranschaulichen die Erfindung. Die Vergleichsbeispiele Nr.41 und 42 zeigen, daß der in der DE-OS 29 22 698 beschriebene Katalysator nur bei kurzen Reaktionszeiten von 1 bis 2 Stunden wirksam ist, während nach 4 Stunden die Aktivität praktisch auf Null abgesunken ist.

Beispiele 1 bis 36

Die Reaktionen werden unter isothermen Bedingungen in einem Rohrreaktor (Wendel 0,6cm, 90cm Länge) in der Gasphase mindestens 6 Stunden lang durchgeführt. Die Trennung und Charakterisierung der Reaktionsprodukte erfolgte nach üblichen Methoden. Die quantitative Bestimmung der Reaktionsprodukte (I) und der Ausgangsstoffe (II) erfolgte gaschromatographisch.

Die in den Beispielen verwendeten Katalysatoren für die Umwandlung von 1,3-Dioxanen zu 4-Oxa-aldehyden sind:

Katalysator A

Der Borzeolith des Pentasiltyps wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit Boehmit im Gewichtsverhältnis 60:40 2-mm-Stränge hergestellt, die bei 110 °C/16 h getrocknet und bei 500 °C/24 h calciniert werden.

Katalysator B

Katalysator B wird erhalten, indem man den reinen Borzeolith des Pentasiltyps (vgl. Herstellung bei Katalysator A) mit Verformungshilfsmitteln zu 2-mm-Strängen verformt, bei 110 °C/16 h trocknet und bei 500 °C/24 h calciniert.

Katalysator C

Katalysator C wird aus einem Eisensilikatzeolith durch Verformung zu Strängen mit Boehmit im Gewichtsverhältnis 60:40 und anschließender Calcination bei 500 °C/16 h hergestellt. Der Eisensilikatzeolith des Pentasil-Typs selbst wird unter hydrothermalen Bedingungen bei autogenem Druck und 165 °C aus 2730 g Wasserglas, gelöst in 2530 g einer wäßrigen 1,6-Hexandiamin-Lösung (Mischung 50:50 Gew.%), und 310 g Eisensulfat, gelöst in 210 g 96 %iger Schwefelsäure und 4250 g Wasser, in einem Rührautoklaven während 4 Tagen synthetisiert, danach abfiltriert, ausgewaschen, bei 100 °C/24 h getrocknet und bei 500 °C/24 h calciniert. Dieser Eisensilikatzeolith hat ein $SiO_2/Fe_2O_3$-Verhältnis von 17,7 und einem $Na_2O$-Gehalt von 0,62 Gew.%. Diese Stränge werden mit 20 %iger Ammoniumchloridlösung solange ionenausgetauscht, bis das bei 500 °C calcinierte Produkt einen Restnatriumgehalt von 0,06 Gew.% aufweist.

Katalysator D

Katalysator A wird mit einer wäßrigen gesättigten $H_2WO_4$-Lösung ca. 30 Minuten getränkt und das Wasser der überstehenden Restlösung wird am Rotationsverdampfer abgezogen. Danach wird der Katalysator bei 130 °C getrocknet und bei 550 °C calciniert. Der Vorgang wird - wenn nötig - wiederholt, bis der Katalysator einen W-Gehalt von 4 Gew.% aufweist.

Katalysator E (Vergleichskatalysator DOS 29 22 698)

51 g eines handelsüblichen $SiO_2$ mit der in DOS 29 22 698 angegebenen Spezifikation (z.B. D 11/11® der BASF) wird mit einer Lösung aus 51 g 0,1 n $CH_3COOH$, 3,19 g $Pr(NO_3)_3.5\ H_2O$, 3,21 g $Nd(NO_3)_3.5\ H_2O$ und 2,66 g $CH_3COOK$ imprägniert, getrocknet und bei 600 °C/4 h calciniert.

In den Beispielen 1 bis 36 werden Umsätze und Selektivitäten sowie die Art des Katalysators, die gewählte Temperatur und Belastung (WHSV) für die erfindungsgemäße Umwandlung von 1,3-Dioxanen zu 4-Oxa-aldehyden beschrieben. Die Versuchsergebnisse sind in der Tabelle 3 zusammengefaßt:
Einsatzstoffe entsprechend der Formel (II)

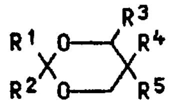 (II),

in der $R^2$ und $R^3$ für Wasserstoff stehen und $R^4$ und $R^5$ eine Methylgruppe darstellen, sind in Tabelle 1 aufgeführt:

Tabelle 1

Edukte der allgemeinen Formel

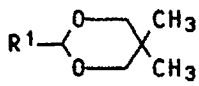

| Nr. | $-R^1$ | Nr. | $-R^1$ |
|---|---|---|---|
| I | $-H$ | XIV | 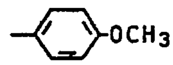 |
| II | $-CH_3$ | XV | 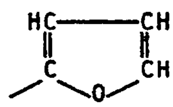 |
| III | $-n-C_3H_7$ | XVI | 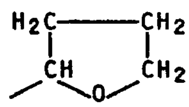 |
| IV | $-i-C_3H_7$ | XVII | 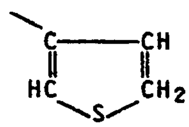 |
| V | 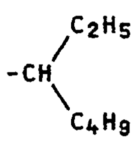 | XVIII | 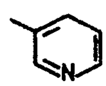 |
| VI | $-CH=CH_2$ | | |
| VII | 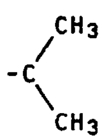 | | |
| VIII | 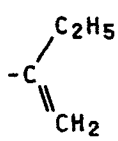 | | |

| Nr. | $-R^1$ | Nr. | $-R^1$ |
|---|---|---|---|
| IX | 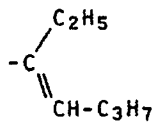 | XIX | $-C(CH_3)_2CH_2OC_4H_9$ |
| X | $-C(CH_3)_2CH=CH_2$ | XX | $-(CH_2)_4-COOCH_3$ |
| XI | $-n-C_9H_{19}$ | | |
| XII | 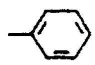 | XXI | 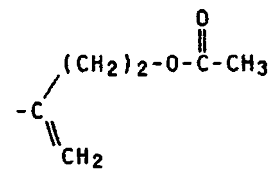 |
| XIII | 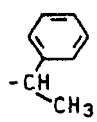 | | |

Weitere Einsatzstoffe entsprechend der Formel (II)

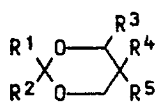

(II),

sind in Tabelle 2 aufgeführt.

Tabelle 2

Edukte der allgemeinen Formel

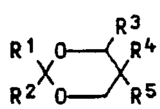

| Nr. | $-R^1$ | $-R^2$ | $-R^3$ | $-R^4$ | $-R^5$ |
|---|---|---|---|---|---|
| XXII | $-n-C_3H_7$ | -H | -H | $-CH_3$ | $-C_2H_5$ |
| XXIII | $-n-C_3H_7$ | -H | -H | $-CH_3$ | $-C_6H_5$ |
| XXIV | $-n-C_3H_7$ | -H | $-i-C_3H_7$ | $-CH_3$ | $-CH_3$ |
| XXV | 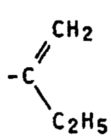 | -H | -H | -H | -H |
| XXVI | $-CH_3$ | $-C_2H_5$ | -H | $-CH_3$ | $-CH_3$ |

Tabelle 2 Fortsetzung

| Nr. | $-R^1$ | $-R^2$ | $-R^3$ | $-R^4$ | $-R^5$ |
|---|---|---|---|---|---|
| XXVII[1] | $-CH_2-(CH_2)_3-CH_2-$ | | -H | $-CH_3$ | $-CH_3$ |
| XXVIII | $-CH_3$ | $-CH_2OCH_3$ | -H | $-CH_3$ | $-CH_3$ |

[1] Strukturformel: 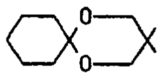

Tabelle 3

| Bsp. | Edukt | Katalysator | Temp. °C | WHSV $h^{-1}$ | Umsatz % | Selektivität 4-Oxaaldehyd % |
|---|---|---|---|---|---|---|
| 1 | I | A | 350 | 2 | 38,1 | 82,9 |
| 2 | I | B | 400 | 2,5 | 19,1 | 68,0 |
| 3 | I | C | 400 | 2 | 50,0 | 75,0 |
| 4 | I | D | 350 | 2 | 37,0 | 79,0 |
| 5 | II | B | 350 | 2 | 93,3 | 48,6 |
| 6 | II | D | 350 | 2 | 81,1 | 53,6 |
| 7 | III | B | 350 | 2 | 85 | 81,4 |
| 8 | IV | B | 250 | 1,8 | 43,5 | 91,7 |
| 9 | V | B | 250 | 2 | 22,4 | 96,0 |
| 10 | VI | B | 250 | 2 | 81,6 | 99,0 |
| 11 | VII | B | 350 | 2 | 74,5 | 81,4 |
| 12 | VII | D | 350 | 2 | 42,0 | 85,4 |
| 13 | VIII | B | 350 | 2 | 87,9 | 79,9 |
| 14 | IX | B | 300 | 3 | 85,8 | 92,8 |
| 15 | IX | D | 250 | 3 | 88,4 | 91,0 |
| 16 | X | B | 300 | 2,5 | 29,5 | 90,2 |
| 17 | XI | B | 300 | 2,5 | 100 | 69,7 |
| 18 | XII | B | 350 | 5 | 100 | 89,4 |
| 19 | XIII | B | 300 | 2,5 | 27,4 | 81,8 |
| 20 | XIV | B | 250 | 2,5 | 90,7 | 78,9 |
| 21 | XV | B | 300 | 2,2 | 67,0 | 94,8 |
| 22 | XVI | B | 300 | 2,2 | 12,3 | 82,1 |
| 23 | XVII | B | 250 | 3,5 | 58,7 | 81,1 |
| 24 | XVIII | B | 300 | 2 | 26,6 | 87,0 |
| 25 | XIX | B | 300 | 2 | 31,9 | 90,3 |

Tabelle 3 (Fortsetzung)

| Bsp. | Edukt | Katalysator | Temp. °C | WHSV $h^{-1}$ | Umsatz % | Selektivität 4-Oxa-aldehyd % |
|---|---|---|---|---|---|---|
| 26 | XX | B | 250 | 3 | 18,0 | 89,4 |
| 27 | XX | B | 300 | 3 | 51,2 | 86,1 |
| 28 | XXI | D | 350 | 2 | 47,4 | 62,0 |
| 29 | XXII | B | 250 | 2,5 | 41,1 | 89,8 |
| 30 | XXII | B | 300 | 2,5 | 65,0 | 77,7 |
| 31 | XXIII | B | 300 | 3 | 57,6 | 45,0 |
| 32 | XXIV | B | 250 | 2,5 | 6,5 | 69,2 |
| 33 | XXV | B | 350 | 2 | 52,2 | 33,5 |
| 34 | XXVI | B | 300 | 1,8 | 31,5 | 87,6 |
| 35 | XXVII | B | 250 | 2 | 25,7 | 94,2 |
| 36 | XXVIII | B | 250 | 2 | 54,0 | 52,1 |

Beispiel 37

Verbindung III wird bei 350 °C und WHSV = 2 $h^{-1}$ am Katalysator B in Gegenwart von 2 l/h $N_2$ umgesetzt. Über die gesamte Reaktionszeit von 75 h wird ein 85 %iger Umsatz mit einer Selektivität von 81,4 % an Butoxy-pivalinaldehyd erzielt. Wie aus Tabelle 4 zu ersehen ist, war während dieser Laufzeit keine Desaktivierung des Katalysators feststellbar.

Tabelle 4

| Reaktionszeit | 11 h | 29 h | 35 h | 52 h | 75 h |
|---|---|---|---|---|---|
| 4-Oxa-aldehyd* | 65,0 | 72,3 | 72,3 | 73,8 | 73,5 |

* in Gew.% im Austrag

Beispiel 38

Ein Langzeitversuch über 8 Tage mit unterschiedlicher Temperaturführung zeigt, daß der Umsatz mit steigender Temperatur stark ansteigt und die Selektivität erst oberhalb 300 °C zurückgeht (Tabelle 5). Die Reaktion wurde in einem Reaktor gefüllt mit 50 g Katalysator B bei einem Durchsatz von 150 ml 2-Propyl-5,5-dimethyl-1,3-dioxan (Verbindung III) pro Stunde unter isothermen Bedingungen im Salzbad durchgeführt.

Tabelle 5

| Temperatur | 250°C | 275°C | 300°C | 325°C |
|---|---|---|---|---|
| Reaktionszeit | 2 Tage | 2 Tage | 2 Tage | 2 Tage |
| Umsatz (%) | 28 | 54 | 82 | 91 |
| Selektivität Butoxy-pivalin-aldehyd (%) | 95 | 95 | 85 | 78 |

Beispiele 39 bis 40

Die Beispiele 39 und 40 sind Vergleichsbeispiele mit Vergleichskatalysator E. Die in Tabelle 6 aufgeführten Versuchsergebnisse können nur in den ersten beiden Stunden erzielt werden, dann arbeitet der Katalysator nicht mehr.

Tabelle 6

| Bsp. | Edukt | Katalysator | Temp. °C | WHSV $h^{-1}$ | Umsatz | Selektivität 4-Oxa-aldehyd % |
|---|---|---|---|---|---|---|
| 39 | XII | E | 300 | 2,5 | 48,9 | 77,1 |
| 40 | III | E | 250 | 2,5 | 12,6 | 13,5 |

Tabelle 7

Ausbeuten und Siedepunkte neuer Verbindungen

| Verbindung | Sdp./p °C mbar | isolierte Ausbeute % |
|---|---|---|
| 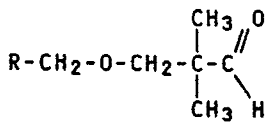 | | |
| mit R- = | | |
| 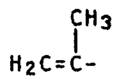 | 105/90 | 58 |
| 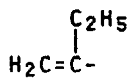 | 87/15 | 69 |
| 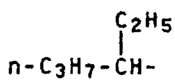 | 85/1 | 77 |
| 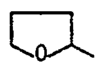 | 96-98/5 | 62 |
| 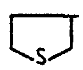 | 92-94/1 | 50 |
| 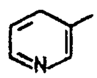 | 103-106/0,6 | 21 |
| $H_3COOC-(CH_2)_4-$ | 110-112/1 | 15 |
| 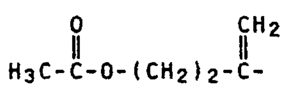 | 94/0,5 | 27 |
| 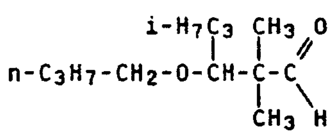 | 83-85/10 | 4 |
| 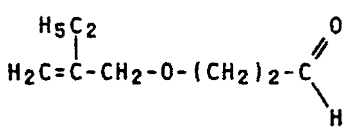 | 74-75/12 | 15 |

## Patentansprüche

**1.** Verfahren zur Herstellung von 4-Oxa-aldehyden der Formel (I)

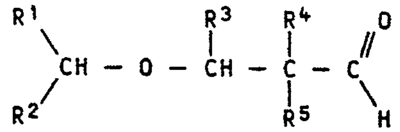

(I)

durch katalytische Isomerisierung von 1,3-Dioxanen, dadurch gekennzeichnet, daß man 1,3-Dioxane der Formel (II)

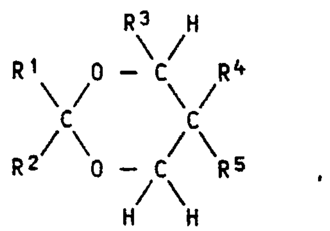

(II),

wobei die Reste $R^1$, $R^2$, $R^4$ und $R^5$ in den Formeln (I) und (II) untereinander gleich oder verschieden sind und für Wasserstoff, geradkettige oder verzweigte Alkyl-, Alkenyl oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder für heterocyclische Reste stehen und darüber hinaus jeweils die Reste $R^1$ und $R^2$ und/oder $R^4$ und $R^5$ zusammen mit dem C-Atom an das sie gebunden sind ein Cycloalkan, Cycloalken oder einen Heterocyclus bilden können, wobei die genannten Reste noch unter den Reaktionsbedingungen inerte Substituenten tragen können und wobei der Rest $R^3$ Wasserstoff oder einen geradkettigen oder verzweigten Alkylrest bedeutet, unter Verwendung von aciden zeolithischen Katalysatoren isomerisiert.

2. Verfahren nach Anspruch 1, dadurch gekennzeichnet, daß Acetale oder Ketale des Propandiols-1,3, des 2-Methyl-, 2,2-Dimethyl-, 2-Methyl-2-Ethyl-, 2-Methyl-2-propyl-, 2-Methyl-2-butyl-, 2-Methyl-2-phenyl-, 1-Isopropyl-2,2-dimethyl-propandiols-1,3 oder des 1,1-Dimethylolcyclohexans oder -pentans isomerisiert werden.

3. Verfahren nach den Ansprüchen 1 und 2, dadurch gekennzeichnet, daß man als Katalysatoren Zeolithe des Pentasiltyps verwendet.

4. Verfahren nach den Ansprüchen 1 bis 3, dadurch gekennzeichnet, daß man als Katalysatoren Aluminiumsilikatzeolithe verwendet.

5. Verfahren nach den Ansprüchen 1 bis 4, dadurch gekennzeichnet, daß man mit Übergangsmetallen dotierte Zeolithe des Pentasiltyps als Katalysatoren verwendet.

6. Verfahren nach den Ansprüchen 1 bis 5, dadurch gekennzeichnet, daß man mit Wolfram dotierte Zeolithe als Katalysatoren verwendet.

7. Verfahren nach den Ansprüchen 1 bis 5, dadurch gekennzeichnet, daß man mit Edelmetallen dotierte Zeolithe als Katalysatoren verwendet.

8. Verfahren nach den Ansprüchen 1 bis 5. dadurch gekennzeichnet, daß man mit seltenen Erdmetallen dotierte Zeolithe als Katalysatoren verwendet.

9. Verfahren nach den Ansprüchen 1 bis 8, dadurch gekennzeichnet, daß man die Zeolithe mit Säuren wie Salzsäure, Flußsäure oder Phosphorsäure behandelt.

10. Verbindungen der Formel (I)

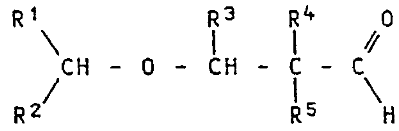

(I),

in der $R^1$ und $R^4$ eine Arylkyl-, Alkylarylgruppe oder einen heterocyclischen Rest bedeutet und $R^2$ und $R^5$ untereinander gleich oder verschieden sind und für Wasserstoff, geradkettige oder verzweigte Alkyl-, Alkenyl- oder Alkinylreste mit bis zu 18 Kohlenstoffatomen, Cycloalkyloder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder für heterocyclische Reste stehen, wobei die genannten Reste noch unter den Reaktionsbedingungen inerte Substituenten tragen können und wobei der Rest $R^3$ Wasserstoff oder einen geradkettigen oder verzweigten Alkylrest bedeutet.

**11.** Verbindungen der Formel (III)

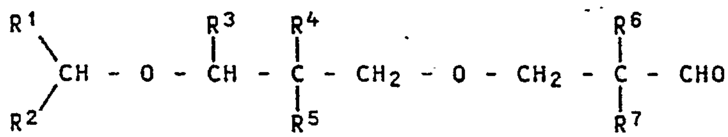

(III)

in der die Reste $R^1$, $R^2$, $R^4$, $R^5$, $R^6$ und $R^7$ untereinander gleich oder verschieden sind und für Wasserstoff, geradkettige oder verzweigte Alkyl-, Alkenyl- oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder für heterocyclische Reste stehen und darüber hinaus jeweils die Reste $R^1$ und $R^2$ und/oder $R^4$ und $R^5$ und/oder $R^6$ und $R^7$ zusammen mit dem C-Atom an das sie gebunden sind ein Cycloalkan, Cycloalken oder einen Heterocyclus mit 5 bis 7 Ringgliedern bilden können und in der der Rest $R^3$ Wasserstoff oder einen geradkettigen oder verzweigten Alkylrest bedeutet.

## Claims

**1.** A process for the preparation of a 4-oxa-aldehyde of the formula (I)

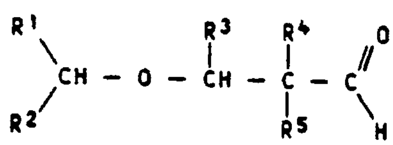

(I)

by catalytic isomerization of a 1,3-dioxane, wherein a I,3-dioxane of the formula (II)

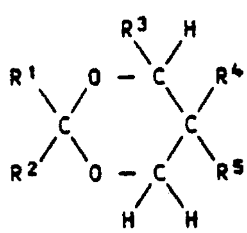

(II)

where $R^1$, $R^2$, $R^4$ and $R^5$ in formulae (I) and (II) are identical or different and are each hydrogen, a straight-chain or branched alkyl, alkenyl or alkinyl radical of not more than 18 carbon atoms, cycloalkyl or cycloalkenyl, each of 5 to 8 carbon atoms, aryl, alkylaryl, aralkyl, aralkenyl or alkenylaryl, each of 6 to 16 carbon atoms, or a heterocyclic radical, and furthermore the radicals $R^1$ and $R^2$ and/or the

radicals $R^4$ and $R^5$, together with the carbon atom to which they are bonded, may form a cycloalkane, a cycloalkene or a heterocyclic structure, and the stated radicals may furthermore carry substituents which are inert under the reaction conditions, and $R^3$ is hydrogen or a straight-chain or branched alkyl radical, is isomerized using an acid zeolite catalyst.

**2.** A process as claimed in claim 1, wherein an acetal or ketal of propane-1,3-diol, of 2-methyl-, 2,2-dimethyl-, 2-methyl-2-ethyl-, 2-methyl-2-propyl-, 2-methyl-2-butyl-, 2-methyl-2-phenyl- or 1-isopropyl-2,2-dimethylpropane-l,3-diol or of 1,1-dimethylolcyclo-hexane or -cyclopentane is isomerized.

**3.** A process as claimed in claim 1 or 2, wherein the catalyst used is a zeolite of the pentasil type.

**4.** A process as claimed in any of claims 1 to 3, wherein the catalyst used is an aluminosilicate zeolite.

**5.** A process as claimed in any of claims 1 to 4, wherein the catalyst used is a zeolite of the pentasil type which is doped with transition metals.

**6.** A process as claimed in any of claims 1 to 5, wherein, the catalyst used is a tungsten-doped zeolite.

**7.** A process as claimed in any of claims 1 to 5, wherein the catalyst used is a zeolite doped with noble metals.

**8.** A process as claimed in any of claims 1 to 5, wherein the catalyst used is a zeolite doped with rare earth metals.

**9.** A process as claimed in any of claims 1 to 8, wherein the zeolite is treated with an acid; which is hydrochloric acid, hydrofluoric acid or phosphoric acid.

**10.** A compound of the formula (I)

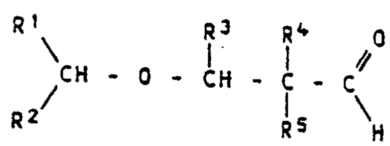

(I)

wherein $R^1$ and $R^4$ are aralkyl, alkylaryl or a heterocyclic radical, and $R^2$ and $R^5$ are identical or different and are each hydrogen, a straight-chain or branched alkyl, alkenyl or alkinyl radical of not more than 18 carbon atoms, cycloalkyl or cycloalkenyl, each of 5 to 8 carbon atoms, aryl, alkylaryl, aralkyl, aralkenyl or alkenylaryl, each of 6 to 16 carbon atoms, or a heterocyclic radical, and the stated radicals may furthermore carry substituents which are inert under the reaction conditions, and $R^3$ is hydrogen or straight-chain or branched alkyl.

**11.** A compound of the formula (III)

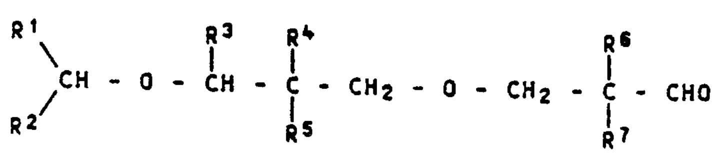

(III)

wherein $R^1$, $R^2$, $R^4$, $R^5$, $R^6$ and $R^7$ are identical or different and are each hydrogen, a straight-chain or branched alkyl, alkenyl or alkinyl radical of not more than 18 carbon atoms, cycloalkyl or cycloalkenyl, each of 5 to 8 carbon atoms, aryl, alkylaryl, aralkyl, aralkenyl or alkenylaryl, each of 6 to 16 carbon atoms, or a heterocyclic radical, and furthermore the radicals $R^1$ and $R^2$ and/or the radicals $R^4$ and $R^5$ and/or the radicals $R^6$ and $R^7$, together with the carbon atom to which they are bonded, may form a cycloalkane, a cycloalkene or heterocyclic structure containing 5 to 7 ring members, and $R^3$ is hydrogen or straight-chain or branched alkyl.

## Revendications

1. Procédé de préparation de 4-oxa-aldéhydes de formule (I)

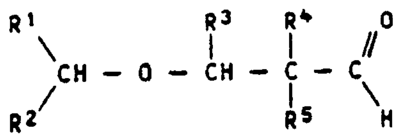

(I)

par isomérisation catalytique de 1,3-dioxannes, caractérisé en ce qu'on isomérise, en utilisant des catalyseurs zéolithiques acides, des dioxannes de formule (II)

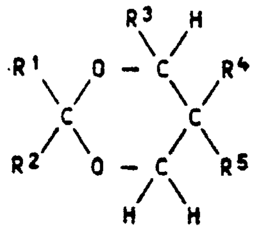

(II),

les restes $R^1$, $R^2$, $R^4$ et $R^5$ dans les formules (I) et (II) étant identiques ou différents et étant mis pour des atomes d'hydrogène, pour des restes alkyle, alcényle ou alcynyle à chaîne droite ou ramifiée renfermant jusqu'à 18 atomes de carbone, pour des restes cycloalkyle ou cycloalcényle à 5-8 atomes de carbone, pour des restes aryle, alkylaryle, aralkyle, aralcényle ou alcénylaryle à 6-16 atomes de carbone ou pour des restes hétérocycliques et, en outre, les restes $R^1$ et $R^2$ et/ou $R^4$ et $R^5$ pouvant respectivement former, avec l'atome de carbone auquel ils sont fixés, un reste cycloalcane, un cycloalcène ou un hétérocycle, les restes cités pouvant encore porter des substituants inertes dans les conditions de la réaction, et le reste $R^3$ représentant un atome d'hydrogène ou un reste alkyle à chaîne droite ou ramifiée.

2. Procédé selon la revendication 1, caractérisé en ce qu'on isomérise des acétals ou des cétals du propanediol-1,3, du 2-méthyl-, 2,2-diméthyl-, 2-méthyl-2-éthyl-,2-méthyl-2-propyl-, 2-méthyl-2-butyl-, 2-méthyl-2-phényl-,1-isopropyl-2,2-diméthyl-propanediol-1,3 ou du 1,1-diméthylolcyclohexane ou -pentane.

3. Procédé selon la revendication 1 ou 2, caractérisé en ce qu'on utilise, comme catalyseurs, des zéolithes du type pentasil.

4. Procédé selon l'une quelconque des revendications 1 à 3, caractérisé en ce qu'on utilise, comme catalyseurs, des zéolithes d'aluminosilicate.

5. Procédé selon l'une quelconque des revendications 1 à 4, caractérisé en ce qu'on utilise, comme catalyseurs, des zéolithes du type pentasildopées avec des métaux de transition.

6. Procédé selon l'une quelconque des revendications 1 à 5, caractérisé en ce qu'on utilise, comme catalyseurs, des zéolithes dopées avec du tungstène.

7. Procédé selon l'une quelconque des revendications 1 à 5, caractérisé en ce qu'on utilise, comme catalyseurs, des zéolithes dopées avec des métaux précieux.

8. Procédé selon l'une quelconque des revendications 1 à 5, caractérisé en ce qu'on utilise, comme catalyseurs, des zéolithes dopées avec des métaux des terres rares.

9. Procédé selon l'une quelconque des revendications 1 à 8, caractérisé en ce qu'on traite les zéolithes par des acides tels que l'acide chlorhydrique, l'acide fluorhydrique ou l'acide phosphorique.

**10.** Composés de formule

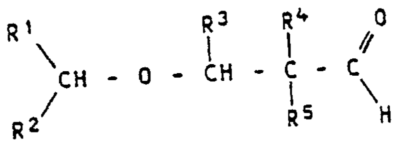

(I),

dans laquelle $R^1$ et $R^4$ représentent chacun un groupement arylalkyle, alkylaryle ou un reste hétérocyclique, et $R^2$ et $R^5$ sont identiques ou différents et sont mis pour des atomes d'hydrogène, pour des restes alkyle, alcényle ou alcynyle à chaîne droite ou ramifiée renfermant jusqu'à 18 atomes de carbone, pour des restes cycloalkyle ou cycloalcényle à 5-8 atomes de carbone, pour des restes aryle, alkylaryle, aralkyle, aralcényle ou alcénylaryle à 6-16 atomes de carbone ou pour des restes hétérocycliques, les restes cités pouvant encore porter des substituants inertes dans les conditions de la réaction, et le reste $R^3$ représente un atome d'hydrogène ou un reste alkyle à chaîne droite ou ramifiée.

**11.** Composés de formule (III)

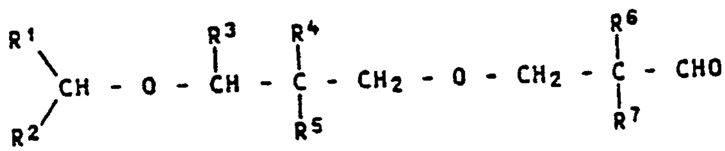

(III)

dans laquelle les restes $R^1$, $R^2$, $R^4$, $R^5$, $R^6$ et $R^7$ sont identiques ou différents et sont mis pour des atomes d'hydrogène, pour des restes alkyle, alcényle ou alcynyle à chaîne droite ou ramifiée renfermant jusqu'à 18 atomes de carbone, pour des restes cycloalkyle ou cycloalcényle à 5-8 atomes de carbone, pour des restes aryle, alkylaryle, aralkyle, aralcényle ou alcénylaryle à 6-16 atomes de carbone ou pour des restes hétérocycliques et, en outre, les restes $R^1$ et $R^2$ et/ou $R^4$ et $R^5$ et/ou $R^6$ et $R^7$ peuvent respectivement former, avec l'atome de carbone auquel ils sont fixés, un reste cycloalcane, cycloalcène ou un hétérocycle à 5-7 chaînons cycliques, et dans laquelle le reste $R^3$ représente un atome d'hydrogène ou un reste alkyle à chaîne droite ou ramifiée.